# EUROPEAN PATENT APPLICATION

(11) **EP 3 628 238 A1**
(43) Date of publication of application: **01.04.2020**
(21) Application number: 18196622.7
(22) Date of filing: 25.09.2018
(51) Int. Cl.: A61B 8/12, A61B 6/12, A61B 6/00, A61B 8/08, A61M 25/00, A61B 8/00

(54) **TORSION CORRECTION FOR INTRAVASCULAR IMAGES**

(71) Applicant: Koninklijke Philips N.V., 5656 AG Eindhoven (NL)
(72) Inventor: ZWAAN, Sergej, 5656 AE Eindhoven (NL); ELENBAAS, Thijs, 5656 AE Eindhoven (NL); DEN HARTOG, Markus Johannes Harmen, 5656 AE Eindhoven (NL); RONGEN, Peter Maria Johannes, 5656 AE Eindhoven (NL); OLIVAN BESCOS, Javier, 5656 AE Eindhoven (NL); TER HORST, Iris, 5656 AE Eindhoven (NL); SPOEL, Willem-Jan, 5656 AE Eindhoven (NL); VAN LAVIEREN, Martijn Anne, 5656 AE Eindhoven (NL)
(74) Representative: de Haan, Poul Erik

(57) **Abstract**

The present invention relates to an imaging device for reconstructing image data of at least one vessel, the device comprising: an input unit; a processing unit; and an output unit; wherein the input unit is configured to receive image data of the vessel, wherein the image data comprises angiographic image data and intravascular image data of the vessel; wherein the processing unit is configured: to perform a spatial co-registration of the intravascular image data with the angiographic image data of the vessel; to perform a rotational registration of at least one side-branch of the vessel in the angiographic image data to a respective side-branch of the vessel in the intravascular image data; to calculate a torsion correction for the intravascular image data of the vessel based on the registered at least one side-branch of the vessel to generate torsion corrected image data of the vessel; and wherein the output unit is configured to provide the torsion corrected image data of the vessel.

## Description

### FIELD OF THE INVENTION

The present invention relates to an imaging device for reconstructing image data of at least one vessel, an according imaging system and method, a computer program element and a computer readable medium.

### BACKGROUND OF THE INVENTION

Coronary arteries supply the heart with blood. In the presence of coronary artery disease vessels are often obstructed (stenosed) by the formation of plaque on the inside of the vessel wall. The patency of the epicardial vessel as well as the ability to provide oxygenated blood to the distal myocardium might be reduced.

A treatment strategy for an obstructed vessel often requires information regarding the plaque structure within the vessel. As identification of plaque can be difficult in angiographic images intravascular ultrasound (IVUS) techniques are often used. An IVUS device often comprises a specially designed catheter with a miniaturized ultrasound module attached to the distal end of the catheter. The IVUS images acquisition process usually provides accurate visualization of the lumen of the vessel, ischemia and the vessel structure.

IVUS image acquisition also provides information about the vessel anatomy. However, creating a correct reconstruction of the vessel can be difficult, since the IVUS device can be twisted during its advancement through the vessel due to intravascular blood flow or friction with the lumen wall of the vessel. The resulting reconstruction of the vessel might thus be distorted, preventing appropriate treatment.

### SUMMARY OF THE INVENTION

There may thus be a need to provide improved IVUS images acquisition.

The object of the present invention is solved by the subject-matter of the independent claims; further embodiments are incorporated in the dependent claims. It should be noted that the following described aspects of the invention apply also for the imaging device, imaging system and the according method.

According to the present invention, an imaging device for reconstructing image data of at least one vessel is provided. The imaging device comprises an input unit, a processing unit and an output unit. The input unit is configured to receive image data of the vessel, wherein the image data comprises angiographic image data and intravascular image data of the vessel. The processing unit is configured to perform a spatial co-registration of the intravascular image data with the angiographic image data of the vessel. The processing unit is configured to perform a rotational registration of at least one side-branch of the vessel in the angiographic image data to a respective side-branch of the vessel in the intravascular image data. Furthermore, the processing unit is configured to calculate a torsion correction for the intravascular image data of the vessel based on the registered at least one side-branch of the vessel to generate torsion corrected image data of the vessel. The output unit is configured to provide the torsion corrected image data of the vessel.

The input unit is configured to receive image data of the vessel, wherein the image data comprises the angiographic image data and the intravascular image data of the vessel. In an example, the angiographic image data is provided by image acquisition techniques such as computed tomography (CT) angiography or magnetic resonance (MR) angiography. In the resulting angiographic image(s) the lumen(s) of the vessel(s) is visualized, e.g. coronary arteries and veins. When using angiography, X-ray based image acquisition is used, e.g. fluoroscopy. A radio-opaque contrast agent might be injected into the vessel(s) allowing their visualization. In examples, 2D (two-dimensional) images of the vessel are provided by the angiographic image data.

The intravascular image data of the vessel is provided by intravascular imaging techniques such as intravascular ultrasound (IVUS), for example. In examples, the intravascular image data can be provided by any 3D modality of which the orientation is unknown. An according intravascular imaging device comprises an ultrasound module or ultrasound probe. The ultrasound module or probe can be provided for example at the distal end of a catheter. The catheter can be a specially designed catheter. The ultrasound module can be a miniaturized ultrasound probe. The IVUS technology enables very accurate visualization of the lumen, ischemia and the vessel structure in the resulting intravascular image. IVUS technology can also be used to define different types of plague provided by the intravascular image which cannot be identified within an angiographic (X-ray) image, for example. In examples, 3D (three-dimensional) images of the vessel are provided by the intravascular image data.

The imaging device comprises a processing unit configured to perform spatial co-registration of the intravascular image data and the angiographic image data of the vessel. Spatial co-registration can be provided by following the location of the intravascular imaging device on the angiographic image during the pullback of the intravascular imaging device. Length and area measurements of the intravascular imaging device on the angiogram can also be provided. In some examples a rough spatial co-registration is sufficient, so that the location, length and area of the imaging device on the angiographic image are provided within a tolerable accuracy.

The processing unit is further configured to perform rotational registration of at least one side-branch of the vessel in the angiographic image data to a respective side-branch of the vessel in the intravascular image data. The rotational registration can comprise an angular difference between the side-branch in the angiographic image and the intravascular image.

The processing unit is configured to calculate a torsion correction for the intravascular image data of the vessel based on the registered at least one side-branch of the vessel to generate torsion corrected image data of the vessel. Thus, the imaging device also enables improved 3D intravascular image data. Furthermore, improved merging of intravascular image data and angiographic image data is provided as well as improved straightened or curved reformats of the vessel.

The torsion correction is calculated for at least one registered side-branch of the vessel. The generated image data comprises torsion corrected intravascular image data. In some examples, the intravascular image data is provided as 3D image data of the vessel taking into account the torsion correction. In other words, the intravascular image data comprises torsion corrected 3D intravascular image data in some examples. In some examples, the torsion correction can be calculated for every co-registered side-branch of the vessel. The torsion correction can also provide a torsion calibration for the intravascular imaging device.

The output unit of the imaging device is configured to provide the torsion corrected image data of the vessel. E.g. the image data can be provided to a display unit for visualizing the torsion corrected and intravascular image of the vessel.

According to the invention, a vessel comprises a coronary vessel, such as coronary arteries and veins. However, also any other kind of vessel is encompassed by the invention. The generated torsion corrected intravascular image data can be provided for different types of vessels.

According to an example, the device further comprises a storage unit configured to provide angiographic image data and/or intravascular image data of the vessel. The storage unit can be provided as external unit or integral unit of the imaging device. In examples, the angiographic image data and/or intravascular image data of the vessel(s) is recorded (measured) and provided to the input unit of the imaging device during the actual image acquisition or after its completion. In some examples, the angiographic image data of the vessel(s) is recorded (measured) before or after the intravascular image data of the respective vessel(s).

According to an example, the angiographic image data of the vessel and the intravascular image data of the vessel are recorded simultaneously. The simultaneously recorded image data for both the angiographic image data and the intravascular image data provides additional temporal co-registration. Both image data can be recorded at the same time. The recording (measuring) could be performed in real-time during the actual image acquisition processes, e.g. during a pullback of the intravascular imaging device. In some examples, X-ray exposure and IVUS measurement are performed simultaneously providing the angiographic image data and intravascular image data of the vessel simultaneously.

According to an example, a longitudinal orientation of an intravascular imaging device is provided based on the torsion correction. Accordingly, also prediction of the movement of the imaging device is provided. The intravascular imaging device provides intravascular image data, e.g. 3D image data, without orientation information. The orientation of the intravascular imaging device is then provided by the longitudinal orientation based on the calculated torsion correction, e.g. the twist or rotation angle along a longitudinal axis of the intravascular imaging device. Thus, artefacts in intravascular images introduced by the torsion of the intravascular imaging device can be corrected. The longitudinal orientation of the intravascular imaging device is determined according to the registered at least one side-branch of the vessel.

According to an example, the rotational registration of the at least one side-branch of the vessel comprises a mapping of the location of the at least one side-branch in the angiographic image data with the location of the respective side-branch in the intravascular image data. In examples, image data of a cross-section of the intravascular volume, in a 3D intravascular image, is taken in the plane of the angiographic image, and the two are mapped (merged).

According to an example, for the rotational registration, at least one parameter of a 2D structure of the vessel is determined based on the angiographic image data and the determined parameter is used for the rotational registration. The 2D structure(s) of the vessel used for rotational registration is preferably unambiguously identified.

According to an example, for the rotational registration, at least one parameter of a 3D structure of the vessel is determined based on the intravascular image data and the determined parameter is used for the rotational registration. The 3D structure(s) of the vessel used for rotational registration is preferably unambiguously identified.

The 2D and 3D structure(s) are identical in both images. In examples, where the angiographic image data is recorded independently from the intravascular image data and is provided as stored angiographic image data to the input unit of the imaging device, the structure(s) may include specific vasculature, distorted vasculature or a significant number of side-branches, e.g. more than three side-branches, so that rotational registration is performed successfully.

According to an example, the torsion correction comprises a torsion correction angle determined by a projection of the 3D structure of the vessel on an image plane provided by the angiographic image data of the vessel. The torsion correction angle can be provided as 2D or 3D torsion correction angle.

Accordingly, an intravascular imaging device, e.g. an ultrasound probe, is rotated with respect to the angiographic image plane, so that the projected 3D structure matches the 2D structure. In other words, the projected 3D structure will match the 2D structure, if the intravascular imaging device is rotated according to the torsion correction angle.

According to an example, the structure of the vessel comprises a bifurcation angle between the vessel and the at least one side-branch or a defining structure of the vessel. The bifurcation angle can be identified easily and unambiguously, as it correlates with the side-branch of the vessel. Other defining structures of the vessel are calcification or plaque, specific distortion of a vessel or other structures suited for unambiguous identification in both angiographic image data and intravascular image data.

According to an example, a projected 3D bifurcation angle, corrected by the torsion correction angle, is substantially equal to the 2D bifurcation angle. In other words, the projected 3D bifurcation angle matches the corresponding 2D bifurcation angle after the torsion correction is applied, e.g. as torsion correction angle.

According to the invention, also an imaging system for reconstructing image data of at least one vessel is provided. The system comprises an imaging device and a display device, wherein the display device is configured to provide the image data of the vessel.

According to the invention, also a method for reconstructing image data of at least one vessel is provided. The method comprising the steps: (a) spatial co-registering of intravascular image data with angiographic image data of the vessel; (b) rotational registering of at least one side-branch of the vessel in the angiographic image data to a respective side-branch of the vessel in the intravascular image data; (c) calculating a torsion correction for the intravascular image data based on the registered at least one side-branch of the vessel. Further, a computer program element for controlling an apparatus such as the imaging device and/or imaging system is provided, which, when being executed by a processing unit, is adapted to perform the method steps. A computer readable medium having stored the program element is provided.

As known from the prior art, IVUS devices can create a 3D reconstruction of a coronary vessel. However, the prior art devices and methods for such reconstruction might be not fully correct because of any torsion of the catheter wire that may have occurred and has not been taken into account.

According to an aspect, by mapping the location of side-branches in the angiogram with the respective side-branch locations in the IVUS measurement, an estimation can be made about the IVUS torsion. This information can be used to improve the 3D reconstruction of the vessel. The use of at least one side-branch of the vessel visible in the angiographic image as well as in the intravascular image provides torsion correction of the reconstruction artefact introduced by a possible twist of an intravascular ultrasound (IVUS) probe during the images acquisition process. The side-branches that are visible in both angiographic images (X-ray images) and IVUS images are used to correct for the torsion induced reconstruction artefacts. One of the applications is to take a cross-section of the IVUS volume (3D intravascular image) in the plane of the X-ray image (angiographic image) and merge the two. This may show e.g. calcifications and plaque during the X-ray based live procedure. Another potential use is to create a correct curved or straightened reformat. Yet another application is in combination with steerable devices. Knowing in which direction with relation to the patient a steerable device will move, could be difficult using prior art IVUS devices, as the orientation of the IVUS with relation to the patient is not known. This provides orientation information of the intravascular imaging device along with prediction of its movement. This applies to any 3D modality of which the orientation is unknown. The torsion correction may also be used for live calibration of optical shape sensing to X-ray.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of an imaging device for reconstructing image data of at least one vessel;
Fig. 2 shows an example of an intravascular and angiographic image overlay;
Fig. 3-7 show examples of a torsion correction for the intravascular image data of a vessel;
Fig. 8 shows an example of an image of a 3D torsion corrected image of a vessel;
Fig. 9 shows an example of an imaging system for reconstructing image data of at least one vessel; and
Fig. 10 shows an example of a flow chart of a method for reconstructing image data of at least one vessel.

### DETAILED DESCRIPTION OF EMBODIMENTS

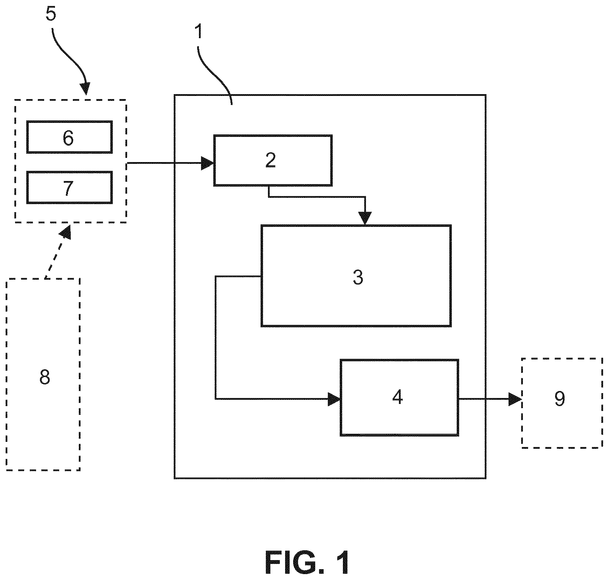

Fig. 1 shows an imaging device 1 comprising an input unit 2, a processing unit 3 and an output unit 4.

The input unit 1 receives image data 5 of a vessel. The image data 5 comprises angiographic image data 6 and intravascular image data 7 of the vessel. The image data 5 comprises angiographic image data 6 and intravascular image data 7 of a coronary vessel.

The processing unit 3 is configured: to perform a spatial co-registration of the intravascular image data 7 with the angiographic image data 6 of the vessel; to perform a rotational registration of at least one side-branch of the vessel in the angiographic image data 6 to a respective side-branch of the vessel in the intravascular image data 7; and to calculate a torsion correction for the intravascular image data 7 of the vessel based on the registered at least one side-branch of the vessel to generate torsion corrected image data 9 of the vessel.

The output unit 4 is configured to provide the torsion corrected image data 9 of the vessel. The image data can be provided to a display unit for visualizing the torsion corrected and intravascular image of the vessel.

In another embodiment, the angiographic image data 6 and/or the intravascular image data 7 of the vessel is provided by a storage unit 8. The storage unit 8 can be provided as external unit or integral unit of the imaging device 1.

In this embodiment, the angiographic image data 6 as well as the intravascular image data 7 of the vessel provided to the input unit 2 of the imaging device 1 during the actual image acquisition. In another embodiment, these data 6,7 is stored in the storage unit 8 and later used for torsion correction calculation.

According to another embodiment (not shown in detail), the angiographic image data 6 and the intravascular image data 7 are recorded independently.

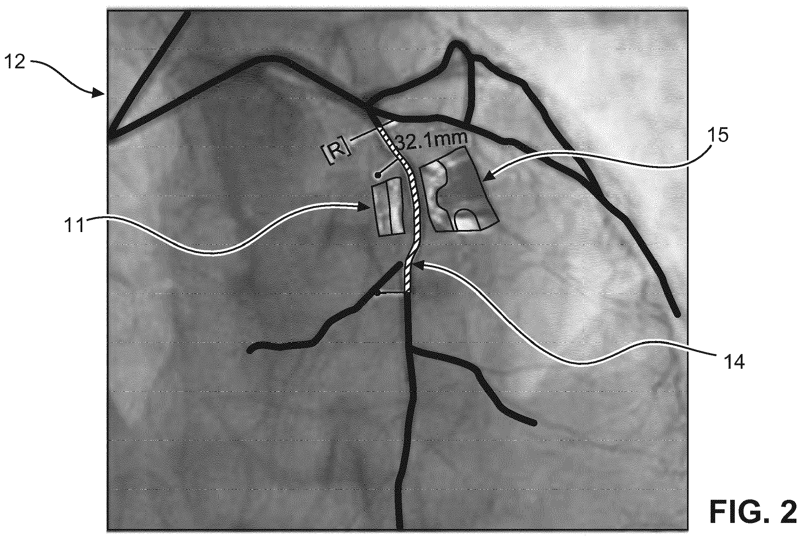

Fig. 2 shows an overlay of an intravascular image 11 and an angiographic image 12. The vessel 14 is shown in the 2D angiographic image 12 and in the intravascular image 15. The intravascular image 15 shows the torsion corrected 3D vessel. If the torsion of the intravascular imaging device provided, e.g. by an IVUS device, it is possible to torsion correct the 3D intravascular image 15 and create the overlay as shown in Fig. 2 on specific locations along the vessel.

In Fig. 2, the specific locations are shown along 32.1 mm of the vessel along with the diameter R of the vessel. Plaque or a stenosis present in the vessel 14 is clearly depicted by the 3D intravascular image 15 but hardly visible in the angiographic image 12 alone. The visibility of the plaque or stenosis will become more apparent as described with reference to the following figures.

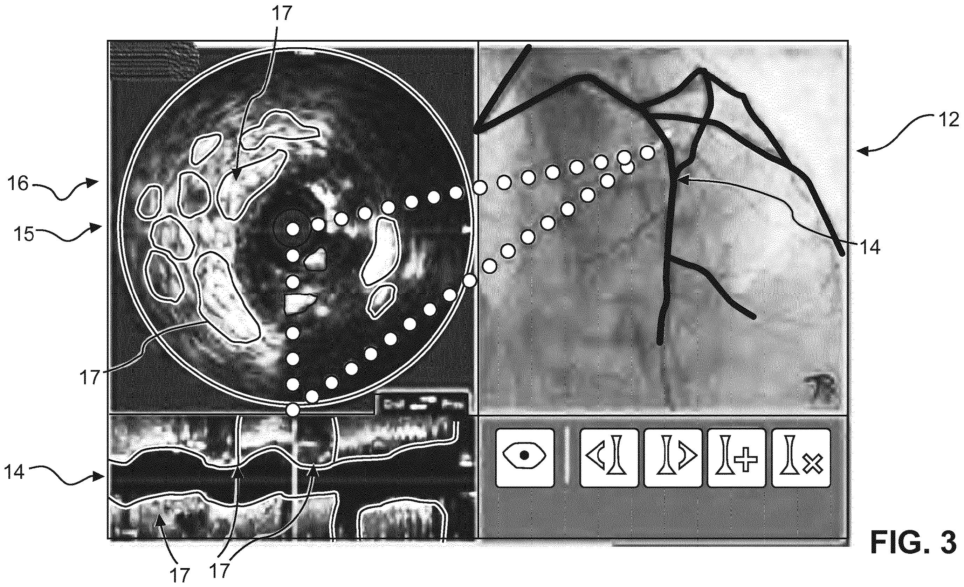

The torsion correction for the intravascular image data of a vessel is described in more detail in Figs. 3 to 7. The angiographic images 12 and the intravascular images 15 of a vessel shown in the figures are provided by the respective image data.

Fig. 3 shows an angiographic image 12 comprising the vessel 14. The lumen 16 of the vessel is shown as a 3D intravascular image 15. The dotted line indicates the co-registration by mapping the location of the 3D intravascular image 15 in the angiographic image 12. The 3D intravascular image 15 is shown as a cross-sectional area of the lumen 16 (upper left part of the Fig. 3) and along the vessel 14 (lower left part of Fig. 3). Along the vessel, the plaque sections 17 of the vessel 14 can be identified and are also visible in the cross-sectional area of the lumen 16. The diameter R of the vessel 14 is also shown in the lower left part of Fig. 3.

In this embodiment, the angiographic image data and the intravascular image data are measured during a pullback of a (not shown) intravascular imaging device. The image data measurement for both, angiographic 12 and intravascular 15 image, is performed during X-ray exposure.

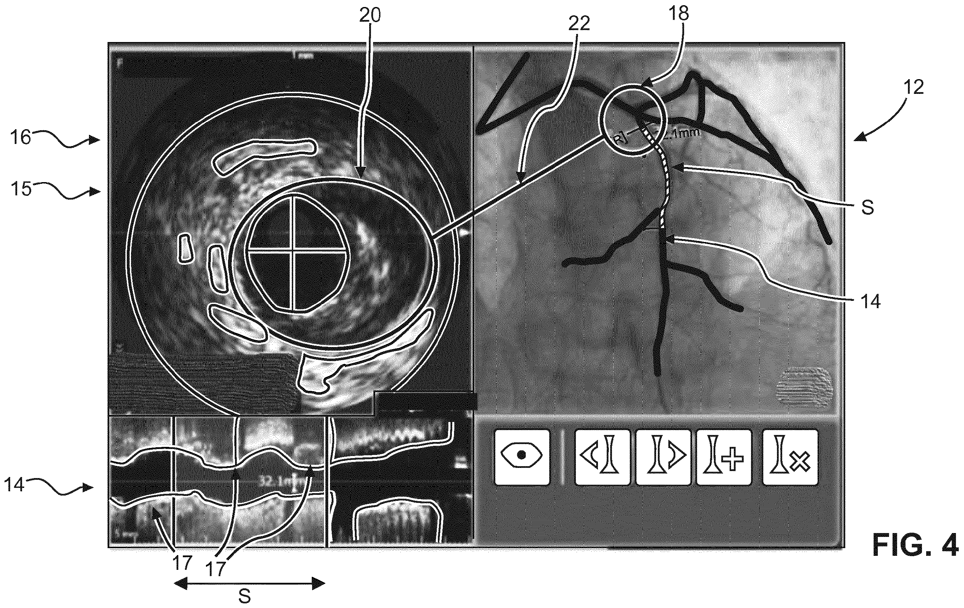

Fig. 4 shows the spatial co-registration of the images of Fig. 3. Due to the measurements having been done simultaneously (cf. description of Fig. 3), a temporal co-registration is provided. During the pullback of the intravascular imaging device the location of the probe (e.g. ultrasound probe) of the intravascular imaging device is followed in the angiographic image 12 along the vessel 14. Thus, spatial co-registration of the angiographic image data and intravascular image data (provided by the angiographic 12 and intravascular 15 image) is performed.

The spatial co-registration is performed along a section S of 32.1 mm of the vessel 14. The same section S is also shown in the angiographic image 12. The mapping of the two images providing the spatial co-registration of the respective image data is explained for one end of section S. A circle 18 provided in the angiographic image 12 indicates the area of the cross-section of the intravascular volume of the lumen 16 shown as circle 20 in the intravascular image 15: the 3D intravascular image 15 is taken in the plane of the angiographic image, and the two are mapped (merged). The mapping is indicated by line 22 connecting the two circles 18, 20.

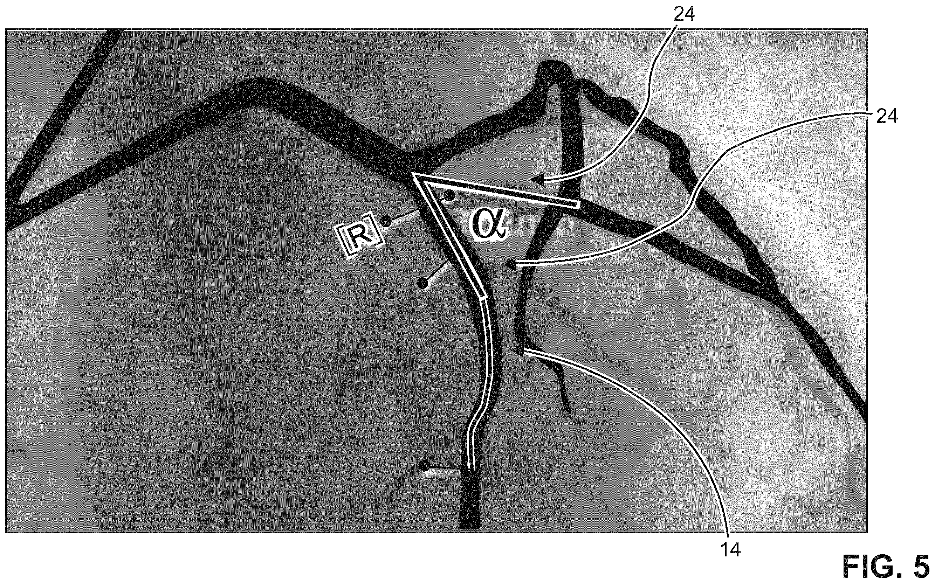

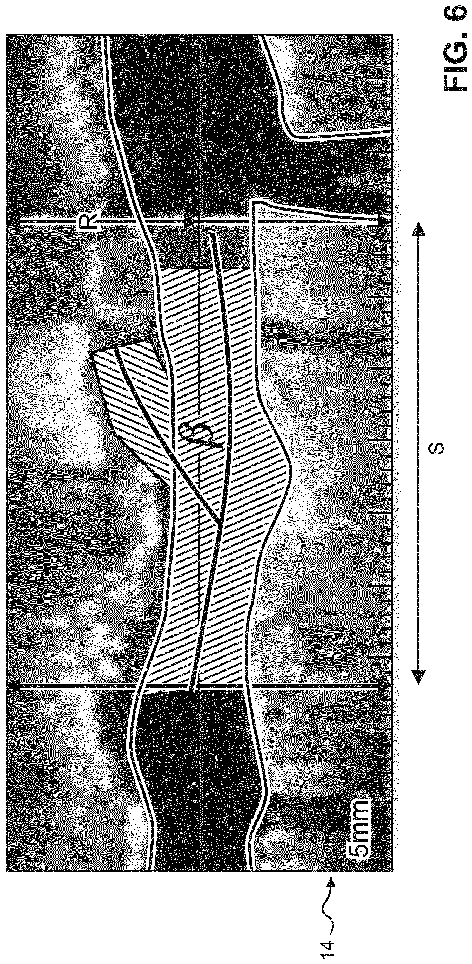

Fig. 5 shows the rotational registration according to an example. The side branches 24 in the angiogram are rotationally registered to the side-branches in the intravascular image. The rotational registration is performed for every side branch found in both the angiographic image 12 and the corresponding intravascular image 15 (shown in Fig. 6). For each correctly registered side-branch 24 a longitudinal orientation of the intravascular imaging device (not shown) can be calculated.

The 2D bifurcation angle α between the vessel 14 and the side-branch 24 of the vessel 14 is shown. In other embodiments, also other defining structures of the vessel 14 can be used instead.

As shown in Fig. 6, the 3D bifurcation angle β is also measured in the intravascular volume. The bifurcation angle β indicates the 3D angle between the vessel and a side-branch as provided by the intravascular imaging device, e.g. an IVUS device. In Fig. 6 at least one side-branch is present for the depicted vessel along section S of the vessel. The diameter R of the vessel is also shown along with plaque present in the vessel.

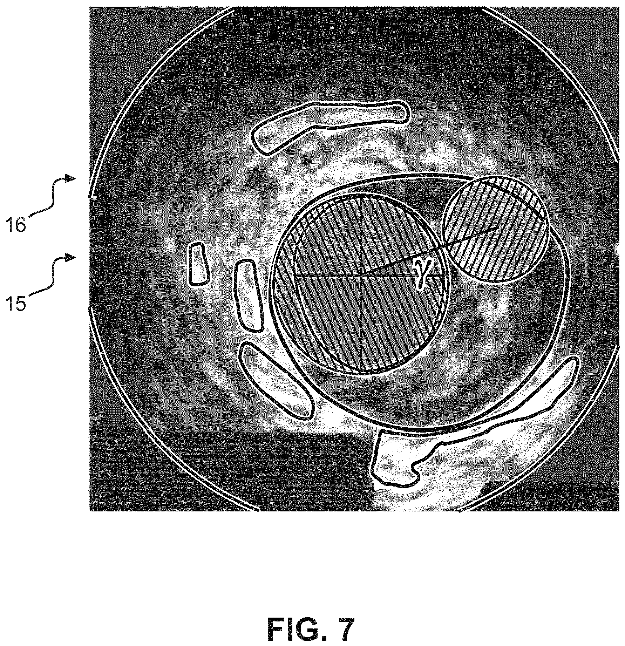

Fig. 7 shows the 3D lumen 16 of the intravascular image 15. As shown in Fig. 7 the 2D angle of the intravascular bifurcation is calculated as projected on the X-ray plane of the angiographic image 12. The intravascular imaging device is then rotated by the torsion correction angle γ with respect to the X-ray plane such that the projected bifurcation angle matches the 2D bifurcation angle α. Thus, a torsion correction is calculated for every co-registered side-branch so that a 3D vessel is provided taking the torsion correction into account.

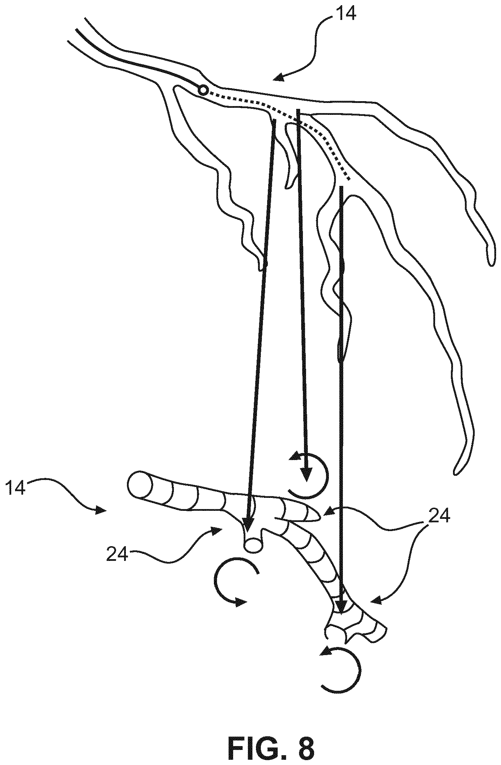

Fig. 8 shows a torsion corrected 3D image of the vessel 14. An enlarged view of the vessel 14 is shown in the lower part of Fig. 8. Arrows indicate the torsion correction based on the respective side-branches 24 of the vessel 14. The dotted line indicates the location of the intravascular imaging device within the vessel during pullback.

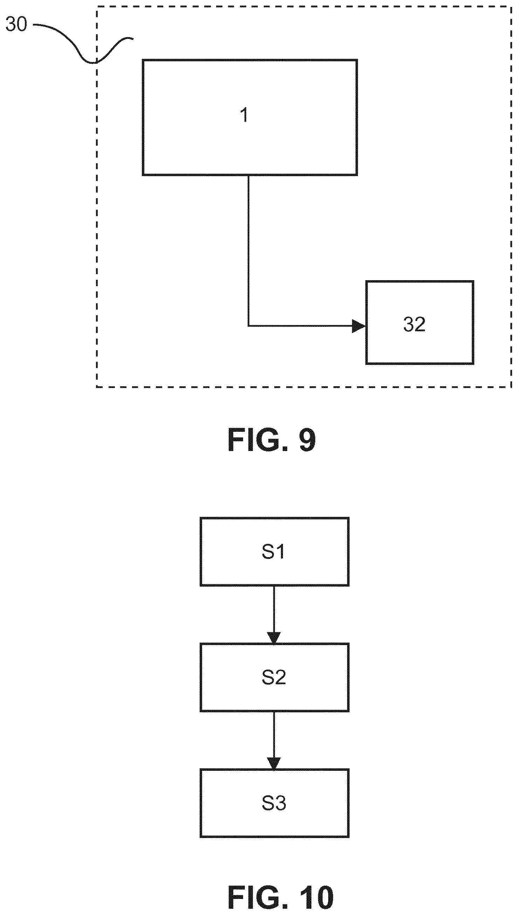

Fig. 9 shows a system 30 for reconstructing image data of a vessel. The system comprises an imaging device 1 and a display device 32. The display device 1 is configured to provide the image data of the vessel as shown in Figure 8. The display device is also configured to show the images as described in Figs 2 to 7.

Fig. 10 shows a flow chart of a method. In an embodiment, the method steps are as described with reference to Figs. 3 to 8.

Accordingly, Fig. 10 shows a flow chart of a method for reconstructing image data of at least one vessel. In step S1 spatial co-registering of intravascular image data with angiographic image data of the vessel is provided. Next, step S2, rotational registering of at least one side-branch of the vessel in the angiographic image data to a respective side-branch of the vessel in the intravascular image data is performed. The next step S3 provides calculating a torsion correction for the intravascular image data based on the registered at least one side-branch of the vessel.

The rotational registration is performed for every side branch found in both the angiographic image data and the corresponding intravascular image data.

In some embodiments, the angiographic image data and intravascular image data of the vessel are recorded simultaneously.

In another example, a computer program or a computer program element is provided that is characterized by being adapted to execute the method steps of the method according to one of the preceding embodiments, on an appropriate system.

The computer program element might therefore be stored on a computer unit, which might also be part of an example. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method.

This exemplary embodiment covers both, a computer program that right from the beginning uses the invention and a computer program that by an up-date turns an existing program into a program that uses the invention.

Further on, the computer program element might be able to provide all necessary steps to fulfil the procedure of an exemplary embodiment of the method as described above.

According to a further example, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further example, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An imaging device for reconstructing image data of at least one vessel, the device comprising:
- an input unit;
- a processing unit; and
- an output unit;
wherein the input unit is configured to receive image data of the vessel, wherein the image data comprises angiographic image data and intravascular image data of the vessel;
wherein the processing unit is configured: to perform a spatial co-registration of the intravascular image data with the angiographic image data of the vessel; to perform a rotational registration of at least one side-branch of the vessel in the angiographic image data to a respective side-branch of the vessel in the intravascular image data; to calculate a torsion correction for the intravascular image data of the vessel based on the registered at least one side-branch of the vessel to generate torsion corrected image data of the vessel; and
wherein the output unit is configured to provide the torsion corrected image data of the vessel.

2. Device according to claim 1, further comprising a storage unit configured to provide the angiographic image data and/or the intravascular image data of the vessel.

3. Device according to claim 1 or 2, wherein a longitudinal orientation of an intravascular imaging device is provided based on the torsion correction.

4. Device according to one of the previous claims, wherein the rotational registration of the at least one side-branch of the vessel comprises a mapping of the location of the at least one side-branch in the angiographic image data with the location of the respective side-branch in the intravascular image data.

5. Device according to one of the previous claims, wherein, for the rotational registration, at least one parameter of a 2D structure of the vessel is determined based on the angiographic image data and the determined parameter is used for the rotational registration.

6. Device according to one of the previous claims, wherein, for the rotational registration, at least one parameter of a 3D structure of the vessel is determined based on the intravascular image data and the determined parameter is used for the rotational registration.

7. Device according to one of the previous claims, wherein the torsion correction comprises a torsion correction angle determined by a projection of the 3D structure of the vessel on an image plane provided by the angiographic image data of the vessel.

8. Device according to one of the previous claims, wherein the structure of the vessel comprises a defining structure of the vessel or a bifurcation angle between the vessel and the at least one side-branch.

9. Device according to claim 8, wherein a projected 3D bifurcation angle, corrected by the torsion correction angle, is substantially equal to the 2D bifurcation angle.

10. An imaging system for reconstructing image data of at least one vessel, the system comprising:
- an imaging device according to one of the previous claims; and
- a display device;
wherein the display device is configured to provide the torsion corrected image data of the vessel.

11. A method for reconstructing image data of at least one vessel, the method comprising the following steps:
a) spatial co-registering of intravascular image data with angiographic image data of the vessel;
b) rotational registering of at least one side-branch of the vessel in the angiographic image data to a respective side-branch of the vessel in the intravascular image data;
c) calculating a torsion correction for the intravascular image data based on the registered at least one side-branch of the vessel.

12. Method according to claim 11, wherein the angiographic image data and intravascular image data of the vessel are recorded simultaneously.

13. A computer program element for controlling an apparatus according to one of the claims 1 to 10, which, when being executed by a processing unit, is adapted to perform the method steps of claim 11 or 12.

14. A computer readable medium having stored the program element of claim 13.
